(19) Europäisches Patentamt
European Patent Office
Office européen des brevets

(11) EP 2 070 903 A1

(12) EUROPEAN PATENT APPLICATION

(43) Date of publication:
17.06.2009 Bulletin 2009/25

(51) Int Cl.:
*C07C 41/02* (2006.01) *C07C 209/18* (2006.01)

(21) Application number: 07380350.4

(22) Date of filing: 12.12.2007

(84) Designated Contracting States:
AT BE BG CH CY CZ DE DK EE ES FI FR GB GR HU IE IS IT LI LT LU LV MC MT NL PL PT RO SE SI SK TR
Designated Extension States:
AL BA HR MK RS

(71) Applicant: Laboratorios del Dr. Esteve S.A.
08041 Barcelona (ES)

(72) Inventors:
- Heller, Detleff
18057 Rostock (DE)
- Preetz, Angelika
18059 Rostock (DE)
- Torrens, Jover, Antoni
08221 Terrasa
(Barcelona) (ES)
- Garcia Lopez, Monica
08018 Barcelona (ES)
- Drexler, Hans-Joachim
18198 Gross - Schwass (DE)

(74) Representative: Bernardo Noriega, Francisco
ABG Patentes, S.L.
Avenida de Burgos, 16D
Edificio Euromor
28036 Madrid (ES)

(54) **Microwave-assisted ring opening reaction**

(57) The present invention is directed to a ring opening reaction of heteronorbomenes and other α,β-unsaturated compounds of formula (Ia) and (Ib):

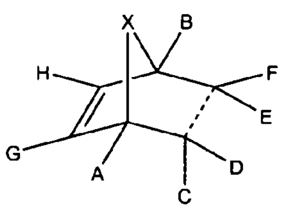

(Ia)

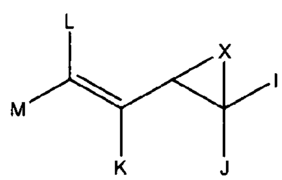

(Ib)

with a nucleophile in the presence of a transition metal complex, characterized in that it is microwave assisted.

EP 2 070 903 A1

## Description

### FIELD OF THE INVENTION

The present invention is directed to microwave-assisted ring opening reactions of heteronorbornenes and other $\alpha,\beta$-unsaturated compounds.

### BACKGROUNG OF THE INVENTION

The first reports on the use of microwave heating to accelerate organic chemical transformations were disclosed in 1986 [Gedye, R. Tetrahedron Lett. 1986, 27, 279-282; Guiguere, R. J. et al. Tetrahedron Lett. 1986, 27, 4945 - 4958]. The initial slow uptake of the technology in the late 1980s and early 1990s has been attributed to its lack of controllability and reproducibility, coupled with a general lack of understanding of the basics of microwave dielectric heating. The risks associated with the flammability of organic solvents in a microwave field and the lack of available systems for adequate temperature and pressure controls were major concerns. Nevertheless, since the late 1990s a myriad of articles have been published in the area of microwave-assisted organic synthesis and it is thought that in few years most chemists will probably use microwave energy to heat chemical reactions.
Although most of the early pioneering experiments in microwave assisted organic synthesis were performed in domestic, sometimes modified, kitchen microwave ovens, the current trend is to use dedicated instruments which have only become available in the last few years for chemical synthesis. Not only is direct microwave heating able to reduce chemical reaction times from hours to minutes, but it is also known to reduce side reactions, increase yields, and improve reproducibility.
A large number of review articles [Kappe, C. O. Angew. Chem. Int. Ed. 2004, 43, 6250; Larhed, M. Acc. Chem. Res. 2002, 35, 717-727], and several books [Microwaves in Organic Synthesis (Ed.: A. Loupy),Wiley-VCH, Weinheim, 2002; Microwave-Assisted Organic Synthesis (Eds.: P. LidstrUm, J. P. Tierney), Blackwell, Oxford, 2004] provide extensive coverage of the subject and are incorporated as reference to the present invention.

On the other hand, the efficient construction of stereochemically complex carbocyclic compounds through the ring opening of heterobicyclic alkenes has become an important reaction for C-C and C-X bond formation. [Lautens, M. Synlett 1993, 177-185. Chiu, P.; Lautens, M. Top. Curr. Chem. 1997, 190, 1-85. Lautens, M.; Fagnou, K.; Hiebert, S. Acc. Chem. Res. 2003, 36, 48-58].
Particular attention has been placed on the desymmetrization of oxobenzonorbornadiene **1,** as the products are precursors to the medicinally important tetrahydronaphthalene moiety [Snyder, S. E.; Aviles-Garay, F. A.; Chakraborti, R.; Nichols, D. E.; Watts, V. J.; Mailman, R. B. J. Med. Chem. 1995, 38, 2395- 2409. Kamal, A.; Gayatri, N. L. Tetrahedron Lett. 1996, 37, 3359- 3362. Kim, K.; Guo, Y.; Sulikowski, G. A. J. Org. Chem. 1995, 60, 6866. Perrone, R.; Berardi, F.; Colabufo, N. A.; Leopoldo, M.; Tortorella, V.; Fiorentini, F.; Olgiati, V.; Ghiglieri, A.; Govoni, S. J. Med. Chem. 1995, 3, 8, 942-949].

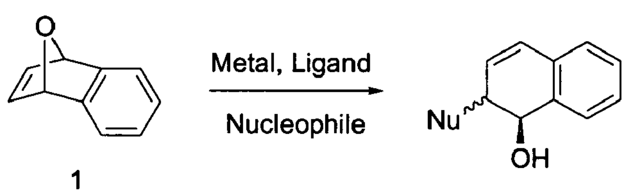


1

The following scheme shows the huge synthetic potential of oxabenzonorbornadiene **1.**

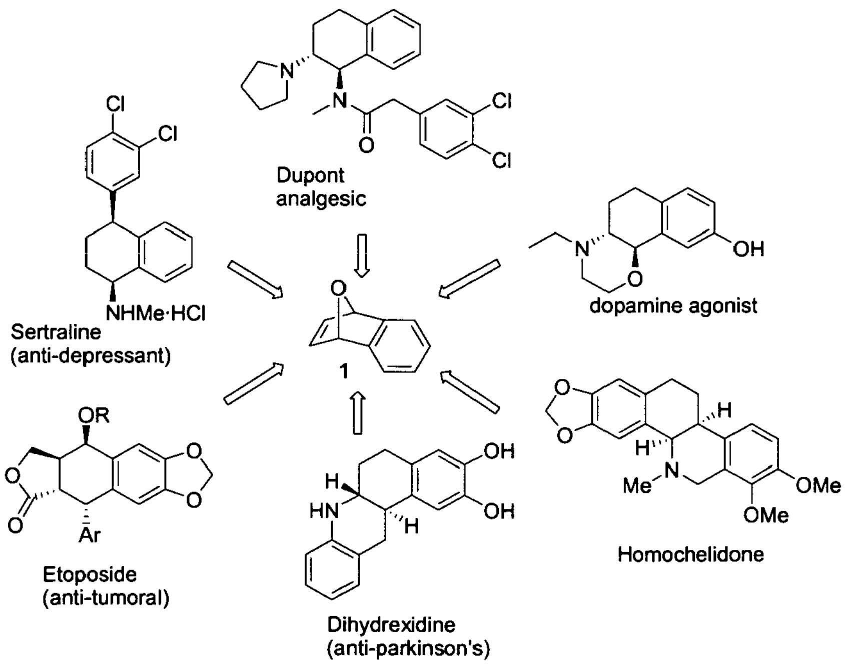

Dupont analgesic
Sertraline (anti-depressant)
dopamine agonist
1
Etoposide (anti-tumoral)
Dihydrexidine (anti-parkinson's)
Homochelidone

WO2001030734 (Fagnou, K.; Lautens, M.) discloses a procedure for making an enantiomerically enriched compound containing a hydronaphthalene ring structure. The process involves reacting oxabenzonorbornadienes compounds with nucleophiles using rhodium as a catalyst and in the presence of a phosphine ligand. The compounds synthesized may be used in pharmaceutical preparations. The catalyst used in this document is $[Rh(COD)Cl]_2$/PPF-$^tBu_2$. Nevertheless, Lautens disclosed later a halide exchange protocol in order to achive better activity and enantioselectivity, specially for other than alcohols or phenolic nucleophiles [Lautens, M.; Fagnou, K.; Yang, D. J. Am. Chem. Soc. 2003, 125, 14884]. Even though the new catalyst, [RhI(PPF-$^tBu_2$)], improved the efficiency of such reactions, high temperatures (always 80 °C or above) were still required.

On the other hand, EP 1 225 166 (Degussa AG) is directed to enantiomerically enriched *N*-acylated β-aminoacids synthesized by catalytic enantioselective hydrogenation of *E*-isomers and *Z*-isomers of 3-amino acrylic acid derivatives in the presence of a pre-catalyst such as $[Rh(MeDuPHOS)COD]BF_4$. The inventors propose this pre-catalyst is first converted to a solvent complex ($[Rh(MeDuPHOS)(MeOH)_2]BF_4$) which is actually the catalytically active specie by prehydrogenation of the diolefinic ligand.

Heller and co-workers have also explored the asymmetric hydrogenation of prochiral substrates in presence of Rh (diolefin) complexes with chiral phosphines. These complexes are hydrogenated in parallel to the asymmetric reaction obtaining thus the true catalytic species, $[Rh(chiral\ diphosphine)(MeOH)_2]BF_4$ [Tetrahedron Lett. 2001, 42, 223; J. Organomet. Chem. 2001, 621, 89; Dalton Trans. 2003, 1606].

In spite of the catalysts and process developed for ring opening reactions, it would be highly desirable to develop new procedures which overcome the problems raised in this type of reactions. In particular, lower reaction temperatures together with lower amounts of substrates would facilitate the industrial application of these processes.

## BRIEF DESCRIPTION OF THE INVENTION

The authors of the present invention have surprisingly found that the application of microwave energy in ring opening reactions improves significantly the results obtained when compared to the transformations under thermal conditions previously described in the prior art. In particular, the application of microwave energy to the asymmetric version of this transformation (asymmetric ring opening, ARO) provides higher enantioselectivities and complete conversions whereas it allows lowering the substrate/nucleophile ratio. In addition, such complexes enable lower reaction temperatures and shorter reaction times.

A first aspect of the invention refers to a process for the catalytic ring opening of α,β-unsaturated compounds of formula (Ia) or (Ib):

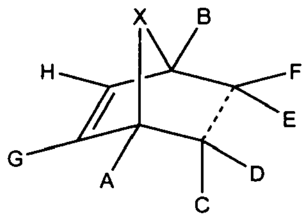

(Ia)

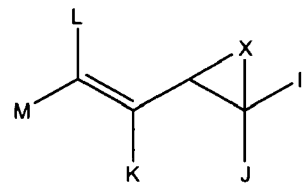

(Ib)

or a stereoisomer, salt or solvate thereof,
wherein the dotted line represents no bond, a single bond or a double bond;
X is oxygen, sulfur or NR, being R hydrogen, substituted or unsubstituted alkyl, substituted or unsubstituted alkenyl, substituted or unsubstituted aryl or a suitable amino protecting group;
A, B, D, F, G, H, J, K and L are each independently selected from the group consisting of hydrogen, substituted or unsubstituted alkyl, substituted or unsubstituted alkenyl, substituted or unsubstituted cycloalkyl, substituted or unsubstituted aryl, substituted or unsubstituted heterocyclyl, substituted or unsubstituted alkoxy, substituted or unsubstituted aryloxy; substituted or unsubstituted alkylamine; substituted or unsubstituted arylamine;
C and E are each independently selected from the group consisting of hydrogen, substituted or unsubstituted alkyl, substituted or unsubstituted alkenyl, substituted or unsubstituted cycloalkyl, substituted or unsubstituted aryl, substituted or unsubstituted heterocyclyl, substituted or unsubstituted alkoxy, substituted or unsubstituted aryloxy; substituted or unsubstituted alkylamine; substituted or unsubstituted arylamine; or when the dotted line represents a single bond, they can be bound together forming a 5-7 member aliphatic or aromatic ring, optionally substituted; wherein in case C and E form an aromatic ring, D and F do not exist;

J and M are each independently selected from the group consisting of hydrogen, substituted or unsubstituted alkyl, substituted or unsubstituted alkenyl, substituted or unsubstituted cycloalkyl, substituted or unsubstituted aryl, substituted or unsubstituted heterocyclyl, substituted or unsubstituted alkoxy, substituted or unsubstituted aryloxy; substituted or unsubstituted alkylamine; substituted or unsubstituted arylamine; or can be bound together forming the compound:

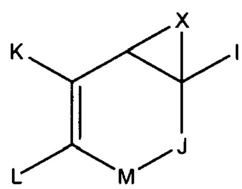

wherein, in this case, J and M are independently selected from substituted or unsubstituted methylene, oxygen, sulfur or NR, being R hydrogen, substituted or unsubstituted alkyl, substituted or unsubstituted alkenyl, substituted or unsubstituted aryl or a suitable amino protecting group; or one of J or M does not exist. with a nucleophile in the presence of a transition metal complex, characterized in that it is microwave assisted.

In a particular embodiment of the invention the transition metal complex used in the ring opening reaction is a rhodium-phosphorus complex selected from:

$[RhX_a(PP)]_2$ and $[Rh(PP)(solv)_2]X_b$

wherein:

PP is a bidentate phosphorus ligand or two monodentate phosphorus ligands;
$X_a$ is a monodentate anionic ligand;
$X_b$ is an anionic counterion; and solv is a coordinating solvent.

Another aspect of the invention is directed to a process for the catalytic ring opening of a compound of formula (Ia'):

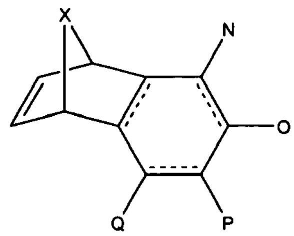

(Ia')

wherein ------ represents a single bond or a double bond, X is oxygen, sulfur or NR, being R hydrogen, substituted or unsubstituted alkyl, substituted or unsubstituted alkenyl, substituted or unsubstituted aryl or a suitable amino protecting group; N, O, P and Q are each independently selected from hydrogen, a substituted or unsubstituted alkyl, a substituted or unsubstituted alkenyl, a substituted or unsubstituted cycloalkyl, a substituted or unsubstituted aryl, a substituted or unsubstituted alkoxy, substituted or unsubstituted aryloxy, substituted or unsubstituted alkylamine; substituted or unsubstituted arylamine, halogen and nitro, with a nucleophile in the presence of a transition metal complex as defined above,
characterized in that the process is microwave assisted.

## DESCRIPTION OF THE DRAWING

Figure 1 shows the $^{31}P$ NMR spectrum of $[Rh(PPF\text{-}P^tBu_2)(THF)_2]BF_4$.

## DETAILED DESCRIPTION OF THE INVENTION

In the context of the present invention, the following terms have the meaning detailed below:

"Alkyl" refers to a straight or branched hydrocarbon chain radical consisting of carbon and hydrogen atoms, containing no unsaturation, having one to eight carbon atoms, and which is attached to the rest of the molecule by a single bond, e. g., methyl, ethyl, n-propyl, i-propyl, n-butyl, t-butyl or n-pentyl. Alkyl radicals may be optionally substituted by one or more substituents such as an aryl, halo, hydroxy, alkoxy, carboxy, cyano, carbonyl, acyl, alkoxycarbonyl, amino, nitro, mercapto or alkylthio.

"Alkenyl" refers to a straight or branched hydrocarbon chain radical consisting of carbon and hydrogen atoms, containing one or more unsaturated bonds, having at least two carbon atoms and which is attached to the rest of the molecule by a single bond, e. g., vinyl or allyl. Alkenyl radicals may be optionally substituted by one or more substituents such as an aryl, halo, hydroxy, alkoxy, carboxy, cyano, carbonyl, acyl, alkoxycarbonyl, amino, nitro, mercapto or alkylthio.

"Cycloalkyl" refers to a stable 3-to 10-membered monocyclic or bicyclic radical which is saturated or partially saturated, and which consist solely of carbon and hydrogen atoms, such as cyclohexyl or adamantyl. Unless otherwise stated specifically in the specification, the term "cycloalkyl" is meant to include cycloalkyl radicals which are optionally substituted by one or more substituents such as alkyl, halo, hydroxy, amino, cyano, nitro, alkoxy, carboxy or alkoxycarbonyl.

"Aryl" refers to single and multiple aromatic hydrocarbon radicals, including multiple ring radicals that contain separate and/or fused aryl groups. Typical aryl groups contain from 1 to 3 separated or fused rings and from 6 to about 18 carbon ring atoms, such as phenyl, naphthyl, indenyl, fenanthryl or anthracyl radical. The aryl radical may be optionally substituted by one or more substituents such as hydroxy, mercapto, halo, alkyl, phenyl, alkoxy, haloalkyl, nitro, cyano, dialkylamino, aminoalkyl, acyl or alkoxycarbonyl.

"Heterocyclyl" refers to a stable 3- to 15- membered ring which consists of carbon atoms and from one to five heteroatoms selected from the group consisting of nitrogen, oxygen, and sulfur, preferably a 4-to 8-membered ring with one or more heteroatoms, more preferably a 5-or 6-membered ring with one or more heteroatoms. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quaternized; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole and tetrahydrofurane.

"Alkoxy" refers to a radical of the formula -ORa where Ra is an alkyl radical as defined above, e. g., methoxy, ethoxy or propoxy. "Aryloxy" refers to a radical of formula -ORb wherein Rb is an aryl radical as defined above.

"Alkylamine" refers to a radical of the formula -NHRa or NRaRb, optionally quaternized, wherein Ra and Rb are independently an alkyl radical as defined above.

"Arylamine" refers to a radical of the formula -NHRa or NRaRb, optionally quaternized, wherein Ra and Rb are independently an aryl radical as defined above.

"Amino protecting group" refers to a group that blocks the $NH_2$ function for further reactions and can be removed under controlled conditions. The amino protecting groups are well known in the art, representative protecting groups are carbamates and amides such as substituted or unsubstituted or substituted acetates. Also different alkyl moeties may serve as amino protecting groups. Additional examples of amino protecting groups can be found in reference books such as Greene and Wuts "Protective Groups in Organic Synthesis", John Wiley & Sons, Inc., New York, 1999.

"Halogen" or "halo" refers to bromo, chloro, iodo or fluoro.

The term "complex" means a molecular structure in which neutral molecules or anions (called ligands) bond to a central metal atom (or ion) by coordinate covalent bonds. Extensive descriptions of terms related to coordination chemistry in reference books such as Robert H. Crabtree "The Organometallic Chemistry of the Transition Metals", Wiley-Interscience; 4 ed., 2005.

The term "catalyst" is recognized in the art and means a substance that increases the rate of a reaction without modifying the overall standard Gibbs energy change in the reaction and without itself being consumed in the reaction. The changing of the reaction rate by use of a catalyst is called catalysis. As used herein, the catalyst is used in a substoichiometric amount relative to a reactant, i. e. a catalytic amount. A preferred catalytic amount is considered herein from 0.0001 to 10 mol% of catalyst relative to the substrate to be opened, more preferably from 0.001 to 1 mol%, more preferably from 0.005 to 0.05 mol% and even more preferably is 0.01 mol%.

The term "ligand" refers to a molecule or ion that is bonded directly (i.e. covalently) to a metal centre.

As used herein in reference to a ligand or metal complex, the term "asymmetric" means that the ligand or complex comprises chiral centres that are not related by a plane or point of symmetry and/or that the ligand or complex comprises an axis of asymmetry due to, for example, restricted rotation, planarity, helicity, molecular knotting or chiral metal complexation.

The term "chiral" refers to molecules which have the property of non superimposability of the mirror image partner.

The term "stereoisomers" refers to compounds which have identical chemical constitution, but differ with regard to the arrangement of the atoms or groups in space.

A "stereoselective process" or an "asymmetric process" is one which produces a particular stereoisomer of a reaction product in preference to other possible stereoisomers of that product.

An "enantioselective reaction" is a reaction that converts an achiral reactant to a chiral, non-racemic product that is enriched in one enantiomer. Enatioselectivity is generally quantified in terms of "enantiomeric excess" ("e. e. "), defined as:

$$e.e. = \left[\frac{(A-B)}{(A+B)}\right] \times 100$$

where A and B are the amounts of enantiomers formed. An enantioselective reaction yields a product with an e.e. greater than zero. Preferred enantioselective reactions yield an e. e. greater than 80%, more preferably greater than 90%, even more preferably greater than 95% and most preferably greater than 98%.

*Nucleophile*

In the context of the present invention, the term "nucleophile" refers to a reagent that forms a chemical bond to its reaction partner (the electrophile) by donating both bonding electrons. Both neutral and anionic nucleophiles are considered in the present invention [for references related to nucleophilicity, please see: Phan T. B.; Breugst, M.; Mayr, H. Angew. Chem. Int. Ed. 2006, 45, 3869-3874. Mayr, H.; Patz, M. Angew. Chem. Int. Ed. Engl. 1994, 33, 938-957].

Non-limiting examples of nucleophiles used in this process are for instance an halogen; a carbon nucleophile selected from 3-indol and activated methylene group; a boronic acid; an oxygen nucleophile selected from water, an alcohol, an ether and a carboxylate; a nitrogen nucleophile selected from ammonia, an amine, an azide, cyanide, isocyanate and isothiocyanate; a sulphur nucleophile selected from a thiol and a thioether; selenocyanate or a phosphine.

Activated methylene groups have electron withdrawing groups in the $\alpha$-position, such as carbonyl or ester groups, such as in acetoacetates.

Preferred nucleophiles are alcohols, ethers and amines.

"Ring opening reaction" is recognized in the art and intended to mean a transition-metal catalyzed process in which a nucleophile reacts with a heterocyclic molecule which has at least a double bond, specifically with a double bond situated in position 2 to a heteroatom, and so the pair of electrons of the double bond is displaced, breaking the heteroatom-carbon bond and thus opening the heterocycle.
As mentioned previously, an aspect of the invention is a process for the catalytic ring opening of α,β-unsaturated compounds of formula (Ia) or (Ib):

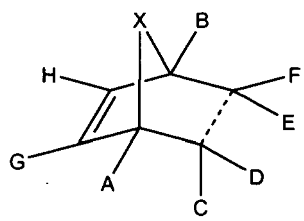
(Ia)

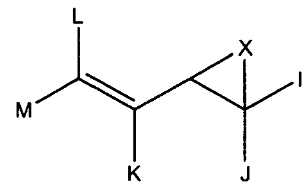
(Ib)

or a stereoisomer, salt or solvate thereof,
wherein the dotted line represents no bond, a single bond or a double bond;
X is oxygen, sulfur or NR, being R hydrogen, substituted or unsubstituted alkyl, substituted or unsubstituted alkenyl, substituted or unsubstituted aryl or a suitable amino protecting group;
A, B, D, F, G, H, J, K and L are each independently selected from the group consisting of hydrogen, substituted or unsubstituted alkyl, substituted or unsubstituted alkenyl, substituted or unsubstituted cycloalkyl, substituted or unsubstituted aryl, substituted or unsubstituted heterocyclyl, substituted or unsubstituted alkoxy, substituted or unsubstituted aryloxy; substituted or unsubstituted alkylamine; substituted or unsubstituted arylamine;
C and E are each independently selected from the group consisting of hydrogen, substituted or unsubstituted alkyl, substituted or unsubstituted alkenyl, substituted or unsubstituted cycloalkyl, substituted or unsubstituted aryl, substituted or unsubstituted heterocyclyl, substituted or unsubstituted alkoxy, substituted or unsubstituted aryloxy; substituted or unsubstituted alkylamine; substituted or unsubstituted arylamine; or when the dotted line represents a single bond, they can be bound together forming a 5-7 member aliphatic or aromatic ring, optionally substituted; wherein in case C and E form an aromatic ring, D and F do not exist;
J and M are each independently selected from the group consisting of hydrogen, substituted or unsubstituted alkyl, substituted or unsubstituted alkenyl, substituted or unsubstituted cycloalkyl, substituted or unsubstituted aryl, substituted or unsubstituted heterocyclyl, substituted or unsubstituted alkoxy, substituted or unsubstituted aryloxy; substituted or unsubstituted alkylamine; substituted or unsubstituted arylamine; or can be bound together forming the compound:

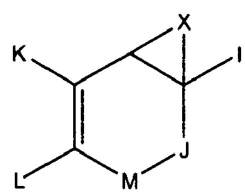

wherein, in this case, J and M are independently selected from substituted or unsubstituted methylene, oxygen, sulfur or NR, being R hydrogen, substituted or unsubstituted alkyl, substituted or unsubstituted alkenyl, substituted or unsubstituted aryl or a suitable amino protecting group; or one of J or M does not exist. with a nucleophile, in the presence of a transition metal complex, characterized in that it is microwave assisted.
In a preferred embodiment of the invention X is oxygen or NR, being R hydrogen, substituted or unsubstituted $(C_1\text{-}C_6)$alkyl, substituted or unsubstituted $(C_1\text{-}C_6)$alkenyl, substituted or unsubstituted phenyl or being the amino group protected as a carbamate, a sulfonamide or with a silyl group.
In another preferred embodiment of the invention A, B, D, F, G, H, J, K and L are each independently selected from the group consisting of hydrogen, substituted or unsubstituted $(C_1\text{-}C_6)$alkyl, substituted or unsubstituted $(C_1\text{-}C_6)$ alkenyl, substituted or unsubstituted $(C_5\text{-}C_6)$cycloalkyl, substituted or unsubstituted phenyl, substituted or unsubstituted heterocyclyl, substituted or unsubstituted $(C_1\text{-}C_6)$alkoxy, substituted or unsubstituted phenoxy; substituted or unsubsti-

tuted ($C_1$-$C_6$)alkylamine; substituted or unsubstituted aniline;

In another preferred embodiment of the invention C and E are each independently selected from the group consisting of hydrogen, substituted or unsubstituted ($C_1$-$C_6$)alkyl, substituted or unsubstituted ($C_1$-$C_6$)alkenyl, substituted or unsubstituted ($C_5$-$C_6$)cycloalkyl, substituted or unsubstituted phenyl, substituted or unsubstituted heterocyclyl, substituted or unsubstituted ($C_1$-$C_6$)alkoxy, substituted or unsubstituted phenoxy; substituted or unsubstituted ($C_1$-$C_6$) alkylamine; substituted or unsubstituted aniline; or when the dotted line represents a single bond, they can be bound together forming a 6 member aliphatic or aromatic ring, optionally substituted; wherein in case C and E form an aromatic ring, D and F do not exist;

In another preferred embodiment of the invention J and M are each independently selected from the group consisting of hydrogen, substituted or unsubstituted ($C_1$-$C_6$)alkyl, substituted or unsubstituted ($C_1$-$C_6$)alkenyl, substituted or unsubstituted ($C_5$-$C_6$)cycloalkyl, substituted or unsubstituted phenyl, substituted or unsubstituted heterocyclyl, substituted or unsubstituted ($C_1$-$C_6$)alkoxy, substituted or unsubstituted phenoxy; substituted or unsubstituted ($C_1$-$C_6$) alkylamine; substituted or unsubstituted aniline; or can be bound together forming the compound:

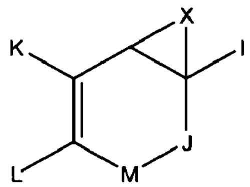

wherein, in this case, J and M are independently selected from substituted or unsubstituted methylene, oxygen, or NR, being R hydrogen, substituted or unsubstituted ($C_1$-$C_6$)alkyl, substituted or unsubstituted ($C_1$-$C_6$)alkenyl, substituted or unsubstituted phenyl or being the amino group protected as a carbamate, a sulfonamide or with a silyl group; or one of J or M does not exist.

In a particular embodiment of the invention, the microwave power applied to the process of the invention varies from 1 to 300W, more preferably from 1 to 200W, more preferably from 1 to 150W, more preferably from 1 to 50W and even more preferably is from about 20 W to about 40 W. Conventional MW generator devices can be used.

In another particular embodiment of the invention, the catalytic ring opening reaction is carried out at a reaction temperature from 15 ˚C to 100 ˚C, more preferably from 25 ˚C to 65 ˚C and even more preferably is from about 45 to about 55 ˚C.

The ring opening reaction is advantageously carried out under an inert protective gas, for example nitrogen or noble gases such as argon.

Likewise, the microwave-assisted reaction is surprisingly carried out in shorter times and lower temperatures as compared with other ring opening reaction described in the prior art. For instance, the ring opening reaction on oxabenzonorbornadiene as substrate takes only 5 minutes at 50 ˚C in getting a full conversion.

Isolation and purification of the opened reaction products can be carried out by known methods, for example precipitation, recrystallization, filtration, extraction, distillation, chromatographic methods or a combination thereof.

In a particular embodiment of the invention the transition metal complex used in the ring opening reaction is a rhodium-phosphorus complex selected from:

$[RhX_a(PP)]_2$ and $[Rh(PP)(solv)_2]X_b$

wherein:

PP is a bidentate phosphorus ligand or two monodentate phosphorus ligands;
$X_a$ is a monodentate anionic ligand;
$X_b$ is an anionic counteiron; and solv is a coordinating solvent.

Conveniently, said complex is preformed *in situ*, that is in the reaction flask. So, when the catalyst used in the ring opening reaction is $[RhX_a(PP)]_2$, this is prepared for example by an halide exchange protocol, as described by Lautens [J. Am. Chem. Soc. 2003, 125, 14884], and when the catalyst is a solvent complex $[Rh(PP)(solv)_2]X_b$, it can be obtained by hydrogenation as described in EP 1 225 166 or in patent application EP 07380... of the same applicant, with title "Rhodium-phosphorus complexes and their use in ring opening reactions" filed on the same day as the present application.

Other reaction conditions such as solvent or different components of the complex that are advantageously employed in ring opening reactions will be comprehensively described below.

*Phosphorus ligand*

Phosphorus ligand represents a ligand covalently bonded to the rhodium by one or two phosphorus atoms. So, both monodentate and bidentate phosphorus ligands are suitable for the present invention. In this sense a "monodentate phosphorus ligand" refers to a molecule containing one phosphorus atom that is covalently bonded to the rhodium, whereas a "bidentate phosphorus ligand" refers to a molecule containing two phosphorus atoms that are covalently bonded to the rhodium. In a preferred embodiment of the invention, the bidentate phosphorus ligand is a diphosphine ligand containing two phosphine groups that are covalently bonded to the rhodium.

The phosphorous ligands used in the present invention are commonly used in organic catalysis by a skilled person. For example, phosphines, phosphinites, phosphonites, phosphites, phosphine-phosphinites, aminophosphines, diaminophosphines are included in the scope of the present invention.
Likewise, both chiral and non-chiral phosphorus ligands are suitable for the present invention.
In a particular embodiment of the invention, the phosphorus ligand is a non-chiral phosphorus ligand. Typical non-chiral phosphorus ligands are $PPh_3$, $P(o\text{-}Tol)_3$, $P(n\text{-}Bu)_3$, $PCy_3$, $P(OEt)_3$, 1,2-bis(diphenylphosphino)ethane (dppe), 1,4-bis (diphenylphosphino)butane (dppb), 1,1'-bis(diphenylphosphino)ferrocene (dppf).

In another particular embodiment, the phosphorus ligand is a chiral phosphorus ligand, preferably a chiral bidentate phosphorus ligand, even more preferably a chiral diphosphine ligand. Handbook of Reagents for Organic Synthesis, Chiral Reagents for Asymmetric Synthesis Leo A. Paquette (Wiley; 1 edition (August 15, 2003) covers a broad list of chiral phosphines, which are herein incorporated by reference. Many chiral diphosphine ligands may be purchased from well-known commercial sources such as Sigma Aldrich or Strem.

More preferably, the chiral diphosphine is selected from BPPFA, Ferrophos, FerroTANE, Josiphos, Mandyphos (Ferriphos), Taniaphos, TRAP, Walphos, BICP, Binap, BPE, BPPM, Chiraphos, Deguphos, Diop, DIPAMP, Duphos, Norphos, Pennphos, Phanephos, PPCP, Prophos, Seguphos, and derivatives thereof. These diphosphine ligands are shown in the following scheme:

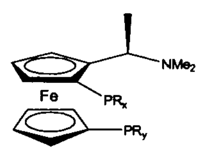

***BPPFA***

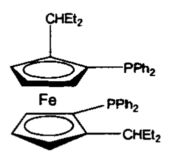

***Ferrophos***

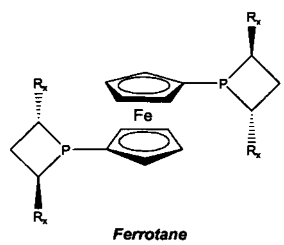

***Ferrotane***

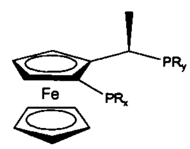

***Josiphos***

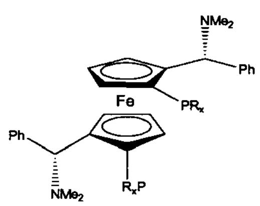

***Mandyphos (Ferriphos)***

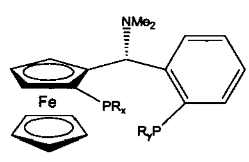

***Taniaphos***

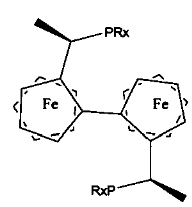

***TRAP***

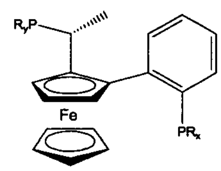

***Walphos***

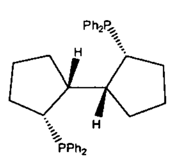

***BICP***

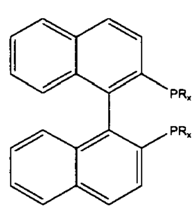

***Binap***

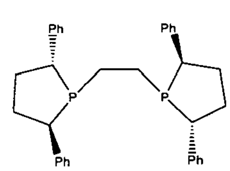

***BPE***

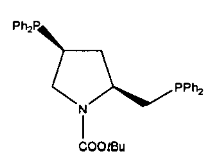

***BPPM***

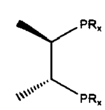

***Chiraphos***

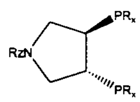

*Deguphos*

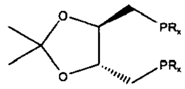

*Diop*

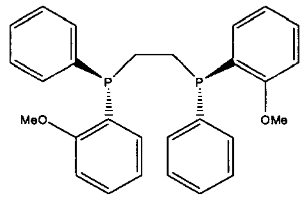

*DIPAMP*

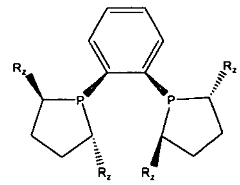

*Duphos*

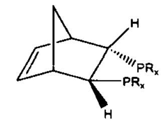

*Norphos*

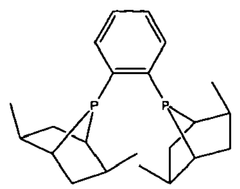

*Pennphos*

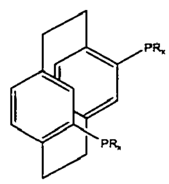

*Phanephos*

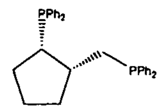

*PPCP*

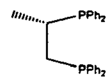

*Prophos*

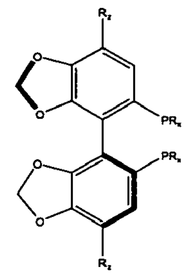

*Segphos*

wherein $R_x$ and $R_y$ are, but not limiting to, substituted or unsubstituted alkyl, such as methyl, ethyl, i-propyl, t-butyl or benzyl; cycloalkyl, such as cyclohexyl; substituted or unsubstituted aryl, such as phenyl, tolyl, 3,5-$(Me)_2$-4-$(MeO)C_6H_2$, 3,5-$(Me)_2C_6H_3$; substituted or unsubstituted heteroaryl, such as 2-furyl.

Examples of these diphosphine ligands include, respectively: N,N-dimethyl-1-[-2,1'-bis(diphenylphosphino) ferrocenyl] ethylamine); 1,1'-bis(diphenylphosphino)-2,2'-bis(1-ethylpropyl) ferrocene; 1,1'-bis[2,4-diethylphosphetano]ferrocene; 1-[2-(diphenylphosphino)ferrocenyl] ethyldicyclohexyl phosphine; 2,2-bis(N,N-dimethylaminophenylmethyl)-1,1-bis (diphenylphosphino) ferrocene; [2-diphenylphosphinoferrocenyl](N,N-dimethylamino)(2-diphenylphosphinophenyl) methane; 2.2'-bis[1-(diphenylphophino)ethyl]-1,1'-biferrocene; 1-[2-(2'-diphenylphosphinophenyl)ferrocenyl]ethyldiphenylphosphine; 2,2'-bis(diphenylphosphino)-1,1'-dicyclopentane; 2,2'-bis(diphenylphosphino)-1,1'-binaphthyl; 1,2-bis(dimethylphospholano)ethane; 2-diphenylphosphinomethyl-4-diphenylphosphino-1-t-butoxycarbonylpyrrolidine; 2,3-bis(diphenylphosphino)butane; 1-benzyl-3,4-bis(diphenylphosphino)pyrrolidine; 2,3-O-isopropylidene-2,3-dihydroxy-1,4-bis-(diphenylphosphino)butane; bis[(2-methoxypheny)phenylphosphino]ethane; 1,2-bis(2,5-dimethylphospholano) benzene; 2,3-bis(diphenylphosphino)-5-norbornene; 1,2-bis(2,5-methyl-7-phosphabicyclo[2.2.1]heptyl)benzene; 4,12-bis(diphenylphosphino)-[2.2]-paracyclophane; 1-(diphenylphosphino)-2-[(diphenylphosphino)methyl]cylopentane. 1,2-bis(diphenylphosphino)propane; 5,5'-bis(diphenylphosphino)-4,4'-bi-1,3-benzodioxole.

In a preferred embodiment of the invention, the diphosphine ligand is a metallocene-type diphosphine ligand. "Metallocene-type diphosphine ligand" means a diphosphine ligand with a metallocene scaffold. A metallocene is an organometallic coordination compound in which one atom of a transition metal is bonded to and only to the face of two cyclopentadienyl [$\eta^5$-$(C_5H_5)$] anions which lie in parallel planes. When the transition metal is iron the metallocene is called ferrocene.

More preferably, the diphosphine ligand is a ferrocene-based diphosphine ligand. In an even preferred embodiment the ferrocene-based diphosphine ligand is selected from the following compounds:

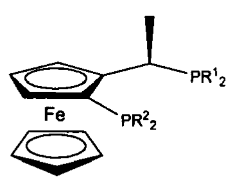

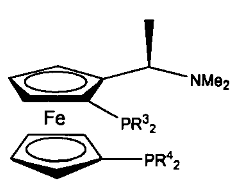

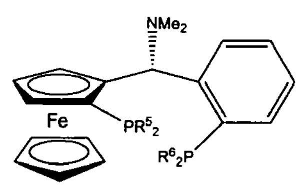

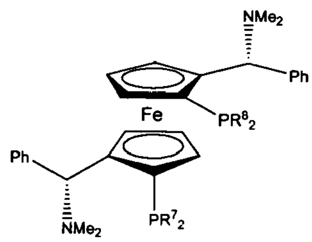

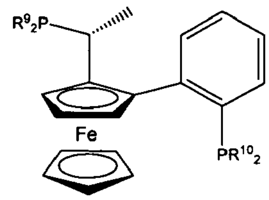

and any stereoisomer, salt or solvate thereof,
wherein
$R^1$ to $R^{10}$ are each independently selected from the group consisting of linear or branched alkyl, sustituted or unsustituted cycloalkyl, sustituted or unsustituted aryl, or substituted or unsubstituted heteroaryl.
Among all the ferrocene-based diphosphine ligands the two following cores are preferred structures:

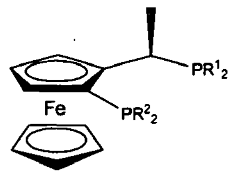

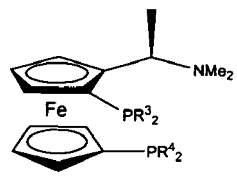

and any stereoisomer, salt or solvate thereof,
wherein $R^1$ to $R^4$ are as defined above for $R^1$ to $R^{10}$.

Even more preferably, the diphosphine ligands are PPF-$P^tBu_2$ and BPPFA

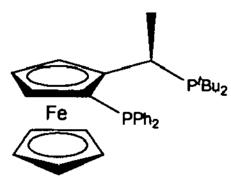

PPF-$P^tBu_2$

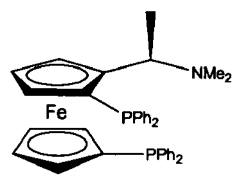

BPPFA

and any stereoisomer, salt or solvate thereof.

*Monodentate anionic ligand*

As "monodentate anionic ligand" it is understood a ligand which forms only one bond with the metal center. In a particular embodiment of the invention the monodentate anionic ligand is selected from fluoride, chloride, bromide, iodide, cyanide, cyanate, isocyanate, $C_1$-$C_3$-carboxylate, sulfonate, phosphonate, carbonate, methylsulfonate, trifluoromethanesulfonate, phenylsulfonate and tosylate.

*Anionic counterion*

As "anionic counterion" it is understood an ionic specie with negative charge that accompanies a cationic transition metal complex, without coordinating to the metal, in order to maintain electric neutrality.
In a particular embodiment of the invention the anionic counterion is selected from $BF_4^-$, $PF_6^-$, $SbF_6^-$, $AsF_6^-$, $ClO_4^-$, $CH_3SO_3^-$, $CF_3SO_3^-$, $HSO_4^-$, $BPh_4^-$ and B[bis-3,5-trifluoromethyl)phenyl]$_4^-$. $BF_4^-$ is the preferred anionic conunterion.

*Coordinating solvent*

A "coordinating solvent" is one which can act as a ligand forming a covalent bond with a transition metal. Typical coordinating solvents are alkanols and ethers, which have atoms with at least one free electron pair through which they coordinate to the transition metal.
The coordinating solvent of the transition metal complex is coordinating to the rhodium by means of an oxygen atom. This solvent is selected from an ether and an alkanol. The ether is preferably selected from tetrahydrofurane, tetrahydropyrane, dioxane, dimethyl ether, diethyl ether, diisopropyl ether, methyl tert-butyl ether and dibutyl ether whereas the alkanol is preferably selected from methanol, ethanol, n-propanol, iso-propanol, n-butanol and tert-butanol. More preferably, the coordinating solvent is tetrahydrofurane or methanol.
Accordingly to the above descriptions, preferred rhodium-phosphorus complexes used in the invention are selected from $[Rh(PPF\text{-}P^tBu_2)(THF)_2]X_b$, $[Rh(BPPFA)(THF)_2]X_b$, $[Rh(PPF\text{-}P^tBu_2)(MeOH)_2]X_b$ and $[Rh(BPPFA)(MeOH)_2]X_b$, wherein $X_b$ is preferably $BF_4$.
In a particular embodiment, the transition metal complex loading is from 0.0001 to 10 mol% with respect to the $\alpha,\beta$-unsaturated compound, more preferably from 0.001 to 1 mol%, more preferably from 0.005 to 0.05 mol% and even more preferably is 0.01 mol%.

*Reaction solvent*

The ring opening reaction is advantageously carried out in the presence of a solvent selected from an ether, an alcohol, a ketone, an ester, an amine, a chlorine-containing solvent, an aromatic solvent, an aprotic polar solvent or mixtures thereof.
In a particular embodiment of the invention the solvent is selected from tetrahydrofurane, tetrahydropyrane, dioxane, dimethyl ether, diethyl ether, dipropyl ether, diisopropyl ether, dibutyl ether, methyl tert-butyl ether, dibenzyl ether, anisol, triethylamine, methanol, ethanol, propanol, isopropanol, butanol, tert-butanol, acetone, ethyl acetate, triethylamine, piperidine, pyridine, tetrachloromethane, dichloromethane, chloroform, 1,2-dichloroethane, benzene, toluene, xylene, dimethylformamide, dimethylacetamide, dimethylsulfoxide, acetonitrile, benzonitrile, nitromethane, propylene carbonate or mixtures thereof.
In another particular embodiment the solvent of the ring opening reaction is also the nucleophile.
Another aspect of the invention is directed to a process for the catalytic ring opening of a compound of formula (Ia'):

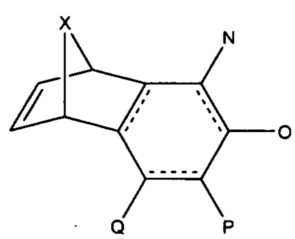

(Ia')

wherein $\overline{- - - - - -}$ represents a single bond or a double bond, X is oxygen, sulfur or NR, being R hydrogen, substituted or unsubstituted alkyl, substituted or unsubstituted alkenyl, substituted or unsubstituted aryl or a suitable amino protecting group; N, O, P and Q are each independently selected from hydrogen, a substituted or unsubstituted alkyl, a substituted or unsubstituted alkenyl, a substituted or unsubstituted cycloalkyl, a substituted or unsubstituted aryl, a substituted or unsubstituted alkoxy, substituted or unsubstituted aryloxy, substituted or unsubstituted alkylamine; substituted or unsubstituted arylamine, halogen and nitro, with a nucleophile in the presence of a rhodium-phosphorus complex selected from: $[RhX_a(PP)]_2$ and $[Rh(PP)(solv)_2]X_b$
wherein PP, Xa, Xb and solv are as defined above,
characterized in that the process is microwave assisted.
In a preferred embodiment N, O, P and Q are independently hydrogen, methoxy or halogen.
In a more preferred embodiment N is hydrogen, methyl, methoxy or halogen, and N, P and O are hydrogen.
The ring opening reaction can be asymmetric or non-asymmetric depending on the presence or absence of chirality in the transition complex used in the reaction. However, in the context of the present invention, it is particularly preferred the execution of an asymmetric ring opening reaction.
A simplified version of the proposed catalytic pathway is the following: firstly, chiral metal complex binds to the heteroatom

and the alkene; afterwards, oxidative insertion of metal catalyst to carbon-heteroatom bond and an $S_N2'$ displacement of the metal catalyst by the nucleophile gives the product and regenerates the catalyst. Nucleophilic attack with inversion provides the product in an $S_N2'$ fashion relative to the metal.

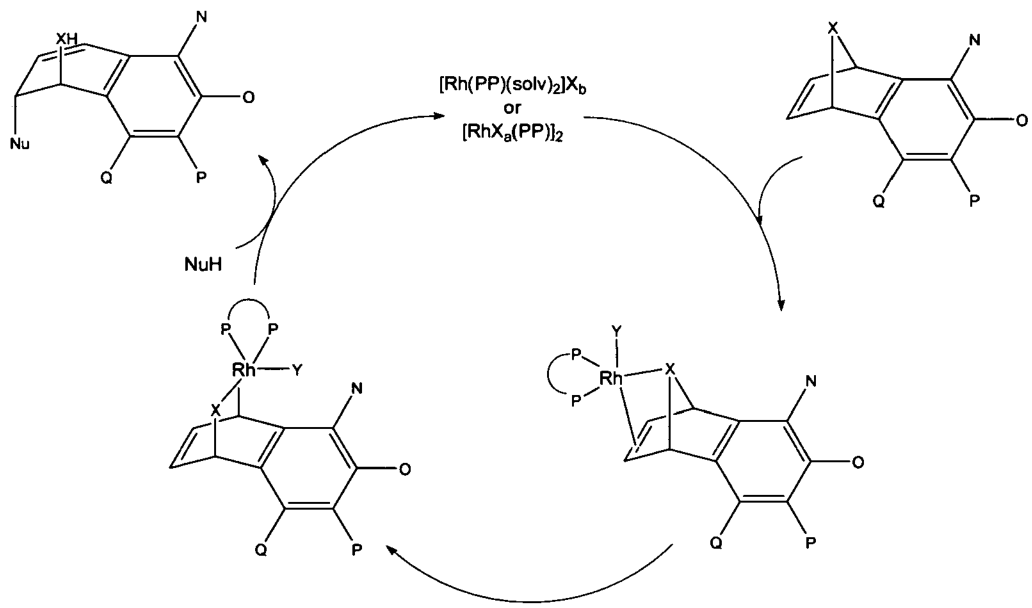


The nucleophile preferably is an alcohol or an amine, more preferably is methanol or dimethylamine.
According to a particular embodiment of the invention, the obtained product of the asymmetric ring opening reaction is selected from:

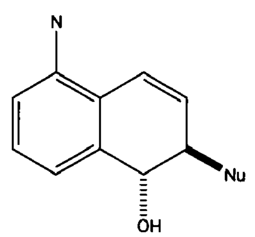 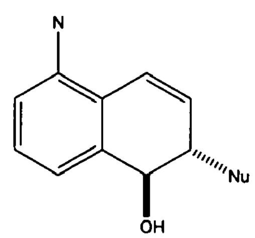 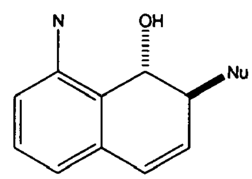 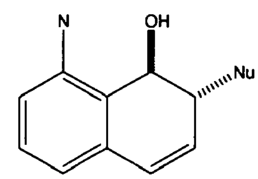

wherein N is hydrogen, methyl, methoxy or halogen; and Nu is a nucleophile selected from an alcohol or an amine, preferably is methanol, dimethylamine or monomethylamine.
The following non-limiting examples will further illustrate specific embodiments of the invention.

**EXAMPLES**

**Example 1-** Synthesis of rhodium-phosphorus complexes

**PPF-P$^t$Bu$_2$**:

[Rh((*S*,*R*)-PPF-P$^t$Bu$_2$)(NBD)]$BF_4$ or [Rh((*S*,*R*)-PPF-P$^t$Bu$_2$)(COD)]$BF_4$ (0.01mm is dissolved in 3 mL of THF-$d_8$ or MeOH-$d_4$ under argon atmosphere. Hydrogen is pressed on the solution, which is then allowed to stir under hydrogen atmosphere for ca. 5 min.

- **[Rh((*S*,*R*)-PPF-P$^t$Bu$_2$)(MeOH)$_2$]$BF_4$**

$^1$H-NMR: 8.59-8.51 (2H, m); 7.67-7.56 (5H, m); 7.46-7.39 (3H, m); 4.96-4.89 (m); 4.63 (1H, br. s); 4.35 (1H, br. s); 4.17 (1H, br. s); 3.81-3.74 (5H, m); 3.39-3.31 (m); 2.88-2.82 (1H, m); 2.00-1.95 (3H, m); 1.71-1.66 (10H, m); 1.34-1.28 (10H, m). $^{31}$P-NNM (in MeOH-$d_4$): 112.3 (J=213.2/54.7 Hz); 49.6 (J=211.5/54.6 Hz)

- **[Rh((*S*,*R*)-PPF-$P^tBu_2$)$(THF)_2$]$BF_4$**

$^1$H-NMR: signals (except for arene protons) covered by solvent signals $^{31}$P-NMR (in THF-$d_8$): 113.2 (J=206.7/54.7 Hz); 51.0 (J=230.2/53.9 Hz)

**DPPF:**

[Rh(DPPF)(NBD)]$BF_4$ or [Rh(DPPF)(COD)]$BF_4$ (0.01 mmol) is dissolved in 3 mL of MeOH-$d_4$ under argon atmosphere. Hydrogen is pressed on the solution, which is then allowed to stir under hydrogen atmosphere for ca. 5 and 45 min, respectively..

- **[Rh(DPPF)$(MeOH)_2$]$BF_4$**

$^1$H-NMR (in MeOH-$d_4$): 7.96-7.89 (8H, m); 7.55-7.41 (12H, m); 4.90 (s); 4.39-4.37 (4H, m); 4.29-4.26 (4H, m); 3.33-3.31 (m). (in NMR also signals of norbornadiene) $^{31}$P-NMR (in MeOH-$d_4$): 54.9 (213.7 Hz).

**Example 2 -** Experimental procedure of thermal ring opening

[Rh((*S*,*R*)-PPF-$P^tBu_2$)(NBD)]$BF_4$ (0.01 mmol) is dissolved in 3 mL of THF under argon atmosphere. Hydrogen is pressed on the solution, which is then allowed to stir under hydrogen atmosphere for ca. 5 min. Hydrogen is exchanged by argon by freezing the solution and securating the gas phase above the solution with argon. This procedure is repeated 3 times. To the cold solvent complex a solution of the substrate (1 mmol) dissolved in 3 ml of THF is added via cannula. The nucleophile (1 mmol) is added to the cool substrate complex solution via syringe. The reaction mixture was then heated at 50°C until the reaction was finished (as judged by TLC or determined separately by HPLC). The solvent was then removed in vacuo and the resulting mixture purified by flash chromatography.

**Example 3** - Experimental procedure of microwave-assisted ring opening

[Rh((*S*,*R*)-PPF-$P^tBu_2$)(NBD)]$BF_4$ (0.01 mmol) is dissolved in 3 mL of THF under argon atmosphere. Hydrogen is pressed on the solution, which is then allowed to stir under hydrogen atmosphere for ca. 5 min. Hydrogen is exchanged by argon by freezing the solution and securating the gas phase above the solution with argon. This procedure is repeated 3 times. To the cold solvent complex a solution of the substrate (1 mmol) dissolved in 3 ml of THF is added via cannula. The substrate complex solution is kept in a cool bath (dry ice/acetone) and stirred for 2 min, and then the nucleophile (1 mmol) is added via syringe to the still cold solution. Via cannula the microwave vial is firstly flushed with argon (ca. 30 s) and secondly filled with the cold reaction solution. (In case of the volatile nucleophile dimethylamine the nucleophile is added directly into the microwave vial with a syringe after addition of the substrate complex.) The reaction will start by heating the solution in the microwave. The reaction mixture was then heated under microwave conditions until the reaction was finished. The solvent was then removed in vacuo and the resulting mixture purified by flash chromatography.

For comparative purposes, other diphosphine complexes of the art have also been tested in the asymmetric ring opening reaction of oxobenzonorbornadiene.

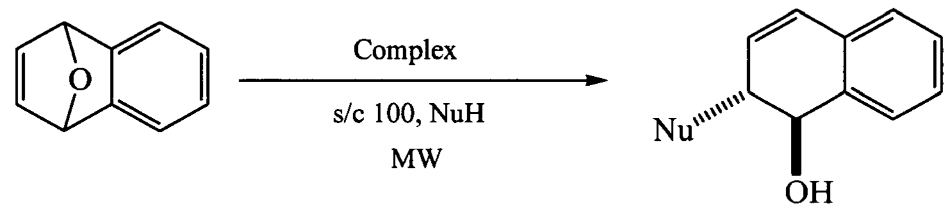


| NuH | ARO conditions | T°C | time (min) | Conv (%) | Yield (%) | ee (%) |
|---|---|---|---|---|---|---|
| MeOH | thermal[a] | 50 | 35 | 100 | 96 | 98.8 |
| | **MICROWAVE assisted[a]** | **50** | **5** | **100** | **96** | **98.0** |
| | thermal[b] | 80 | 900 | 100 | 96 | 97.0 |
| | MICROWAVE assisted[b] | 80 | 30 | 80 | 42 | 95.3 |
| HNMePh | thermal[a] | 50 | 35 | 100 | 95 | 98.0 |
| | **MICROWAVE assisted[a]** | **50** | **5** | **100** | **98** | **96.6** |
| | thermal[c] | 80 | 120 | 100 | 96 | 92.0 |
| | MICROWAVE assisted[b] | 80 | 30 | 59 | 47 | 63.6 |

a) $[Rh((S,R)\text{-PPF-P}^tBu_2)(THF)_2]BF_4$
b) $[RhCl(COD)]_2$ / $((S,R)\text{-PPF-P}^tBu_2)$
c) $[RhI((S,R)\text{-PPF-P}^tBu_2)]_2$ s/c = substrate/catalyst ratio; yield means isolated yield and conv means conversion yield.

As it is shown, the use of microwave energy provides the best results. Preferably in combination with [Rh(PP)$(solv)_2$]$X_b$ as defined above, the transformation runs almost instantly at low temperatures. Also the reaction takes place with complete conversions and excellent enantioselectivities.

## Claims

1. A process for the catalytic ring opening of α,β-unsaturated compounds of formula (Ia) or (Ib):

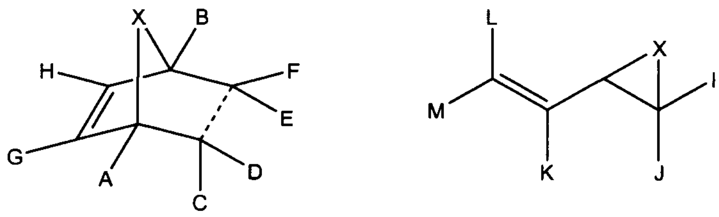

(Ia) (Ib)

or a stereoisomer, salt or solvate thereof,
wherein:
the dotted line represents no bond, a single bond or a double bond;
X is oxygen, sulfur or NR, being R hydrogen, substituted or unsubstituted alkyl, substituted or unsubstituted alkenyl, substituted or unsubstituted aryl or a suitable amino protecting group;
A, B, D, F, G, H, J, K and L are each independently selected from the group consisting of hydrogen, substituted or unsubstituted alkyl, substituted or unsubstituted alkenyl, substituted or unsubstituted cycloalkyl, substituted or unsubstituted aryl, substituted or unsubstituted heterocyclyl, substituted or unsubstituted alkoxy, substituted or unsubstituted aryloxy; substituted or unsubstituted alkylamine; substituted or unsubstituted arylamine; C and E are each independently selected from the group consisting of hydrogen, substituted or unsubstituted alkyl, substituted or unsubstituted alkenyl, substituted or unsubstituted cycloalkyl, substituted or unsubstituted aryl, substituted or unsubstituted heterocyclyl, substituted or unsubstituted alkoxy, substituted or unsubstituted aryloxy; substituted or unsubstituted alkylamine; substituted or unsubstituted arylamine; or when the dotted line represents a single bond, they can be bound together forming a 5-7 member aliphatic or aromatic ring, optionally substituted; wherein in case C and E form an aromatic ring D and F do not exist; J and M are each independently selected from the group consisting of hydrogen, substituted or unsubstituted alkyl, substituted or unsubstituted alkenyl, substituted or unsubstituted cycloalkyl, substituted or unsubstituted aryl, substituted or unsubstituted heterocyclyl, substituted or unsubstituted alkoxy, substituted or unsubstituted aryloxy; substituted or unsubstituted alkylamine; substituted or unsubstituted arylamine; or can be bound together forming the compound:

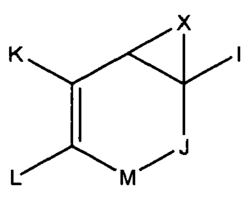

wherein, in this case, J and M are independently selected from substituted or unsubstituted methylene, oxygen, sulfur or NR, being R hydrogen, substituted or unsubstituted alkyl, substituted or unsubstituted alkenyl, substituted or unsubstituted aryl or a suitable amino protecting group; or one of K or I does not exist; with a nucleophile in the presence of a transition metal complex, **characterized in that** it is microwave assisted.

2. The process according to claim 1, wherein the microwave power is from 1 to 300W, more preferably 1 to 200W, more preferably from 1 to 150W, more preferably 1 to 50W and even more preferably is from about 20 to about 40W.

3. The process according to claims 1 or 2, wherein said process is carried out at a reaction temperature from 15 °C

to 100 °C, more preferably from 25 °C to 65 °C and even more preferably is from about 45 to about 55 °C.

**4.** The process according to any of claims 1 to 3 wherein the transition metal complex is a rhodium phosphorus complex selected from:

$$[RhX_a(PP)]_2 \text{ and } [Rh(PP)(solv)_2]X_b$$

wherein:

PP is a bidentate phosphorus ligand or two monodentate phosphorus ligands;
$X_a$ is a monodentate anionic ligand;
$X_b$ is an anionic counteiron; and solv is a coordinating solvent.

**5.** The process according to claim 4 wherein the phosphorus ligand is a chiral phosphorus ligand.

**6.** The process according to claim 5 wherein the chiral phosphorus ligand is a chiral bidentate ligand, preferably a chiral diphosphine ligand.

**7.** The process according to claims 6 wherein the chiral diphosphine ligand is selected from BPPFA, Ferrophos, FerroTANE, Josiphos, Mandyphos (Ferriphos), Taniaphos, TRAP, Walphos, BICP, Binap, BPE, BPPM, Chiraphos, Deguphos, Diop, DIPAMP, Duphos, Norphos, Pennphos, Phanephos, PPCP, Prophos, Segphos and derivatives thereof.

**8.** The process according to claim 6 wherein the diphosphine ligand is a metallocene-type diphosphine ligand.

**9.** The process according to claim 8 wherein the metallocene-type diphosphine ligand is a ferrocene-based diphosphine ligand.

**10.** The process according to claim 9 wherein the ferrocene-based diphosphine ligand is selected from the following compounds:

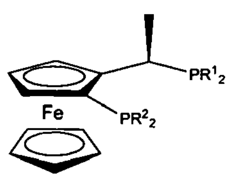
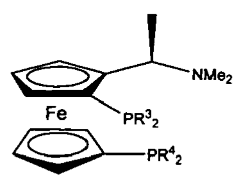
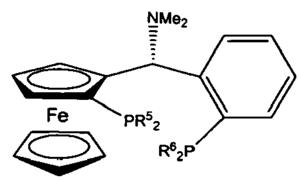
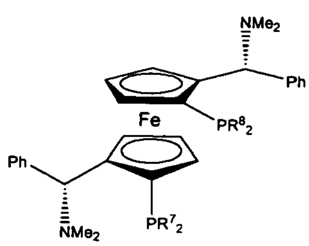
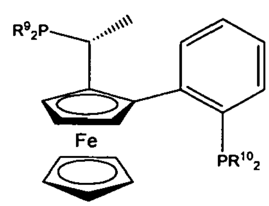

and any stereoisomer, salt or solvate thereof,
wherein $R^1$ to $R^{10}$ are each independently selected from the group consisting of linear or branched alkyl, sustituted or unsustituted cycloalkyl, sustituted or unsustituted aryl, or substituted or unsubstituted heteroaryl.

**11.** The process according to claim 10 wherein the ferrocene-based diphosphine ligand is selected from:

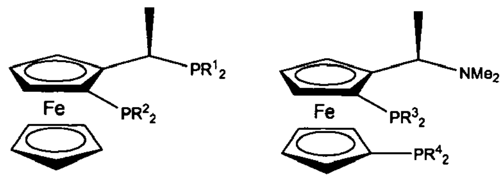

and any stereoisomer, salt or solvate thereof, wherein $R^1$ to $R^4$ are as defined in claim 9.

**12.** The process according to claim 11 wherein the ferrocene-based diphosphine ligand is selected from:

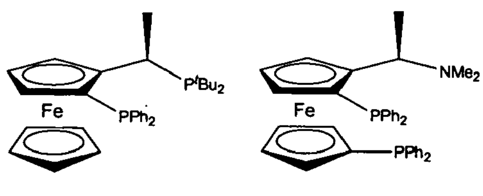

PPF-P$^t$Bu$_2$ BPPFA

and any stereoisomer, salt or solvate thereof.

**13.** The process according to any of claims 4 to 12, wherein $X_a$ is selected from fluoride, chloride, bromide, iodide, cyanide, cyanate, isocyanate, $C_1$-$C_3$-carboxylate, sulfonate, phosphonate, carbonate, methylsulfonate, trifluoromethanesulfonate, phenylsulfonate and tosylate.

**14.** The process according to any of claims 4 to 13, wherein $X_b$ is selected from $BF_4^-$, $PF_6^-$, $SbF_6^-$, $AsF_6^-$, $ClO_4^-$, $CH_3SO_3^-$, $CF_3SO_3^-$, $HSO_4^-$, $BPh_4^-$ and B[bis-(3,5-trifluoromethyl)phenyl]$_4^-$.

**15.** The process according to any of claims 4 to 14 wherein the solvent is selected from an ether and an alkanol.

**16.** The process according to claim 15 wherein the ether is selected from tetrahydrofurane, tetrahydropyrane, dioxane, dimethyl ether, diethyl ether, diisopropil ether, methyl tert-butyl ether and dibutyl ether, and the alkanol is selected from methanol, ethanol, n-propanol, iso-propanol, n-butanol and tert-butanol.

**17.** The process according to any of claims 4 to 16, wherein the rhodium-phosphorus complex is selected from [Rh(PPF-P$^t$Bu$_2$)(THF)$_2$]$X_b$, [Rh(BPPFA)(THF)$_2$]$X_b$, [Rh(PPF-P$^t$Bu$_2$)(MeOH)$_2$]$X_b$ and [Rh(BPPFA)(MeOH)$_2$]$X_b$.

**18.** The process according to claim 17 wherein $X_b$ is $BF_4^-$.

**19.** The process according to any of claims 1 to 18, wherein the transition metal complex loading is from 0.0001 to 10 mol%, more preferably from 0.001 to 1 mol%, more preferably from 0.005 to 0.05 mol% and even more preferably is 0.01 mol%.

**20.** The process according to any of claims 1 to 19 wherein the nucleophile is an halogen; a carbon nucleophile selected from 3-indol and activated methylene group; a boronic acid; an oxygen nucleophile selected from water, an alcohol, an ether and a carboxylate; a nitrogen nucleophile selected from ammonia, an amine, an azide, cyanide, isocyanate and isothiocyanate; a sulphur nucleophile selected from a thiol and a thioether; selenocyanate or a phosphine.

**21.** The process according to any of claims 1 to 20, wherein said process is carried out in the presence of a solvent selected from an ether, an alcohol, a ketone, an ester, an amine, a chlorine-containing solvent, an aromatic solvent, an aprotic polar solvent or mixtures thereof.

**22.** The process according to claim 21, wherein the solvent is selected from tetrahydrofurane, tetrahydropyrane, dioxane, dimethyl ether, diethyl ether, dipropyl ether, diisopropyl ether, dibutyl ether, methyl tert-butyl ether, dibenzyl ether, anisol, triethylamine, methanol, ethanol, propanol, isopropanol, butanol, tert-butanol, acetone, ethyl acetate, triethylamine, piperidine, pyridine, tetrachloromethane, dichloromethane, chloroform, 1,2-dichloroethane, benzene, toluene, xylene, dimethylformamide, dimethylacetamide, dimethylsulfoxide, acetonitrile, benzonitrile, nitromethane, propylene carbonate or mixtures thereof.

**23.** The process according to claims 21 or 22, wherein the solvent is the nucleophile.

**24.** A process for the catalytic ring opening of a compound of formula (Ia'):

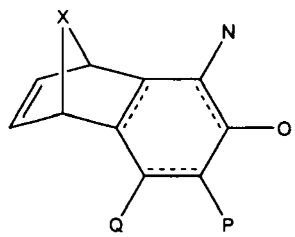

(Ia')

wherein ------ represents a single bond or a double bond, X is oxygen, sulfur or NR, being R hydrogen, substituted or unsubstituted alkyl, substituted or unsubstituted alkenyl, substituted or unsubstituted aryl or a suitable amino protecting group; N, O, P and Q each independently are selected from hydrogen, a substituted or unsubstituted alkyl, a substituted or unsubstituted alkenyl, a substituted or unsubstituted cycloalkyl, a substituted or unsubstituted aryl, a substituted or unsubstituted alkoxy, substituted or unsubstituted aryloxy, substituted or unsubstituted alkylamine, substituted or unsubstituted arylamine, halogen and nitro, with a nucleophile in the presence of a rhodium-phosphorus complex as defined in any of claims 4 to 18, **characterized in that** it is microwave assisted.

**25.** The process according to claim 24 wherein N, O, P and Q are independently hydrogen, methoxy or halogen.

**26.** The process according to claim 25 wherein N is hydrogen, methyl, methoxy or halogen, and O, P and Q are hydrogen.

**27.** The process according to any of claims 24 to 26 wherein the nucleophile is an alcohol or an amine, preferably is methanol or dimethylamine.

**28.** The process according to any of claims 24 to 27 wherein the obtained product is selected from:

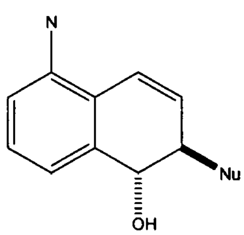 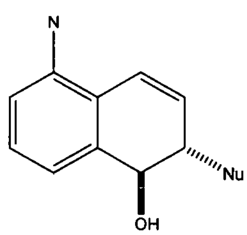 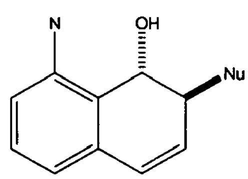 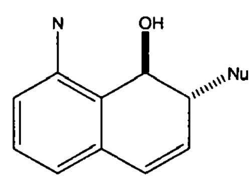

wherein M is hydrogen, methyl, methoxy or halogen; and Nu is a nucleophile selected from an alcohol or an amine, preferably is methanol, dimethylamine or monomethylamine.

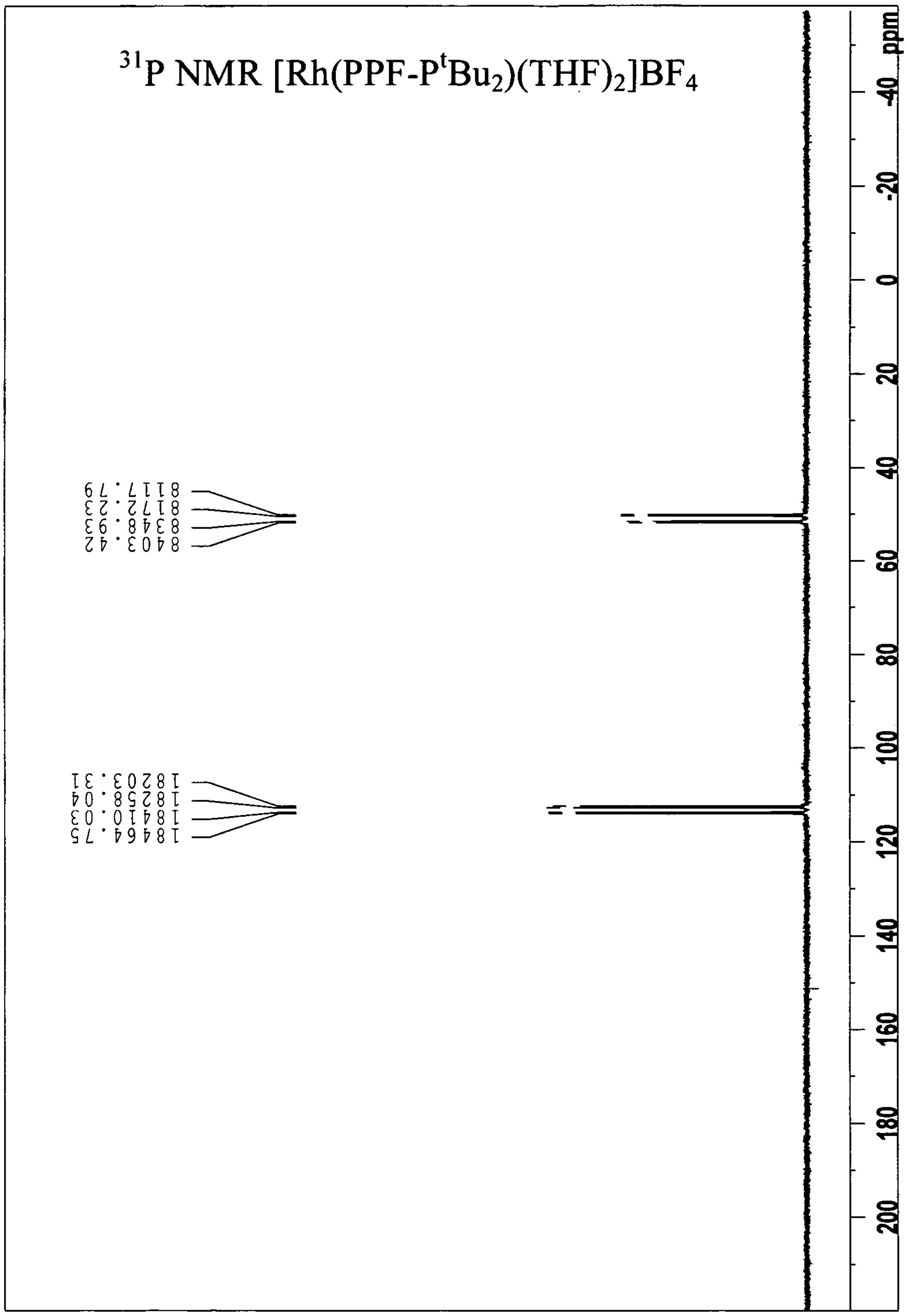
$^{31}P$ NMR $[Rh(PPF\text{-}P^tBu_2)(THF)_2]BF_4$
8117.79
8172.23
8348.93
8403.42
18203.31
18258.04
18410.03
18464.75
ppm

**Fig. 1**

European Patent Office

# EUROPEAN SEARCH REPORT

Application Number
EP 07 38 0350

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KURESHY, SING, KHAN, ABDI, AGRAWAL, MAYANI, JASRA: "Microwave assisted asymmetric ring opening of meso-epoxides with aromatic amines catalyzed by a Ti-S-(-)-BINOL complex" TETRAHEDRON LETTERS, vol. 47, 2006, pages 5277-5279, XP002478993 Scheme 2* table 1 * ----- | 1-23 | INV. C07C41/02 C07C209/18 |
| X | DESAI, D'SOUZA, FOETHER, JOHNSON, LINDSAY: "Regioselectivity in a highly efficient, microwave-assisted epoxide aminolysis" SYNTHESIS, 8 February 2007 (2007-02-08), pages 902-910, XP002478994 Schemes 2, 3 ----- | 1-23 | |
| D,X | LAUTENS MARK ET AL: "Transition metal-catalyzed enantioselective ring-opening reactions of oxabicyclic alkenes" ACCOUNTS OF CHEMICAL RESEARCH, ACS, WASHINGTON, DC, US, vol. 36, no. 1, 1 January 2003 (2003-01-01), pages 48-58, XP009090288 ISSN: 0001-4842 * the whole document * ----- -/-- | 1-28 | TECHNICAL FIELDS SEARCHED (IPC) C07C C07D C07F |

The present search report has been drawn up for all claims

| Place of search | Date of completion of the search | Examiner |
|---|---|---|
| Munich | 2 May 2008 | Pérez Carlón, Raquel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

European Patent Office

# EUROPEAN SEARCH REPORT

Application Number
EP 07 38 0350

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | YAMAMOTO ET AL: "Multi-component coupling synthesis of polycyclic pyrrole derivatives via Ir- and Rh-catalyzed cycloisomerizations with microwave heating"<br>TETRAHEDRON, ELSEVIER SCIENCE PUBLISHERS, AMSTERDAM, NL,<br>vol. 63, no. 41,<br>31 August 2007 (2007-08-31), pages 10149-10160, XP022226163<br>ISSN: 0040-4020<br>* the whole document *<br>----- | 1-28 | |
| | | | TECHNICAL FIELDS SEARCHED (IPC) |

The present search report has been drawn up for all claims

| Place of search | Date of completion of the search | Examiner |
|---|---|---|
| Munich | 2 May 2008 | Pérez Carlón, Raquel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2001030734 A, Fagnou, K.; Lautens, M.
- EP 1225166 A, Degussa AG
- EP 07380 A

### Non-patent literature cited in the description


- **Gedye, R.** *Tetrahedron Lett.,* 1986, vol. 27, 279-282
- **Guiguere, R. J. et al.** *Tetrahedron Lett.,* 1986, vol. 27, 4945-4958
- **Kappe, C. O.** Angew. Chem. 2004, vol. 43, 6250
- **Larhed, M.** *Acc. Chem. Res.,* 2002, vol. 35, 717-727
- Microwaves in Organic Synthesis. Wiley-VCH, 2002
- Microwave-Assisted Organic Synthesis. Blackwell, 2004
- **Lautens, M.** *Synlett,* 1993, 177-185
- **Chiu, P. ; Lautens, M.** *Top. Curr. Chem.,* 1997, vol. 190, 1-85
- **Lautens, M. ; Fagnou, K. ; Hiebert, S.** *Acc. Chem. Res.,* 2003, vol. 36, 48-58
- **Snyder, S. E. ; Aviles-Garay, F. A. ; Chakraborti, R. ; Nichols, D. E. ; Watts, V. J. ; Mailman, R. B.** *J. Med. Chem.,* 1995, vol. 38, 2395-2409
- **Kamal, A. ; Gayatri, N. L.** *Tetrahedron Lett.,* 1996, vol. 37, 3359-3362
- **Kim, K. ; Guo, Y. ; Sulikowski, G. A.** *J. Org. Chem.,* 1995, vol. 60, 6866
- **Perrone, R. ; Berardi, F. ; Colabufo, N. A. ; Leopoldo, M. ; Tortorella, V. ; Fiorentini, F. ; Olgiati, V. ; Ghiglieri, A. ; Govoni, S.** *J. Med. Chem,* 1995, vol. 3 (8), 942-949
- **Lautens, M. ; Fagnou, K. ; Yang, D.** *J. Am. Chem. Soc.,* 2003, vol. 125, 14884
- *Tetrahedron Lett.,* 2001, vol. 42, 223
- *J. Organomet. Chem.,* 2001, vol. 621, 89
- *Dalton Trans.,* 2003, 1606
- **Greene ; Wuts.** Protective Groups in Organic Synthesis. John Wiley & Sons, Inc, 1999
- **Robert H. Crabtree.** The Organometallic Chemistry of the Transition Metals. Wiley-Interscience, 2005
- **Phan T. B. ; Breugst, M. ; Mayr, H.** Angew. Chem. Int. Ed. 2006, vol. 45, 3869-3874
- **Mayr, H. ; Patz, M.** Angew. Chem. Int. Ed. Engl. 1994, vol. 33, 938-957
- **Lautens.** *J. Am. Chem. Soc.,* 2003, vol. 125, 14884
- Chiral Reagents for Asymmetric Synthesis. **Leo A. Paquette.** Handbook of Reagents for Organic Synthesis. Wiley, 15 August 2003